(19) Europäisches Patentamt
European Patent Office
Office européen des brevets

(11) Veröffentlichungsnummer: **0 339 418 B1**

# (12) EUROPÄISCHE PATENTSCHRIFT

(45) Veröffentlichungstag der Patentschrift: **26.07.95**

(51) Int. Cl.6: **C07C 233/00**, A01N 37/24, A01N 53/00, C07D 309/08, C07D 305/08, A01N 43/32, A01N 43/16, A01N 43/20

(21) Anmeldenummer: **89106818.1**

(22) Anmeldetag: **17.04.89**

Die Akte enthält technische Angaben, die nach dem Eingang der Anmeldung eingereicht wurden und die nicht in dieser Patentschrift enthalten sind.

## (54) Substituierte Cycloalkyl- bzw. Heterocyclyl-carbon-säureanilide.

(30) Priorität: **29.04.88 DE 3814505**

(43) Veröffentlichungstag der Anmeldung: **02.11.89 Patentblatt 89/44**

(45) Bekanntmachung des Hinweises auf die Patenterteilung: **26.07.95 Patentblatt 95/30**

(84) Benannte Vertragsstaaten: **BE CH DE ES FR GB IT LI NL**

(56) Entgegenhaltungen:
**EP-A- 0 052 473**
**EP-A- 0 100 615**
**EP-A- 0 116 409**
**EP-A- 0 205 271**
**DE-A- 1 900 202**
**DE-A- 1 907 117**
**GB-A- 1 255 161**
**US-A- 4 184 867**

(73) Patentinhaber: **BAYER AG**
**D-51368 Leverkusen (DE)**

(72) Erfinder: **Krüger, Bernd-Wieland, Dr.**
**Unterboschbach 19**
**D-5060 Bergisch-Gladbach 2 (DE)**
Erfinder: **Sasse, Klaus, Dr.**
**Pützweg 13**
**D-5060 Bergisch-Gladbach 2 (DE)**
Erfinder: **Hagemann, Hermann, Dr.**
**Kandinsky-Strasse 52**
**D-5090 Leverkusen 1 (DE)**
Erfinder: **Marhold, Albrecht, Dr.**
**Carl-Duisberg-Strasse 329**
**D-5090 Leverkusen 1 (DE)**
Erfinder: **Brandes, Wilhelm, Dr.**
**Eichendorffstrasse 3**
**D-5653 Leichlingen (DE)**

**EP 0 339 418 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Cycloalkyl- bzw. Heterocyclyl-carbonsaureanilide, ein Verfahren zu ihrer Herstellung und ihre Verwendung zur Bekämpfung von Schädlingen, insbesondere von Pilzen.

Es ist bekannt, daß bestimmte Phenole gute fungizide Eigenschaften besitzen (vergl. W. Krämer in Pflanzenschutz und Schädlingsbekämpfung, Ed. K.-H. Büchel, S. 143 ff, 1977, Georg Thieme Verlag, Stuttgart, und darin zitierte Literatur).

Weiterhin sind viele Carbonsäureanilide mit fungizider Wirkung, insbesondere mit hoher Wirkung gegen Benzimidazol-tolerante Pflanzenpathogene, bekannt (vergl. EP 117 024, EP 125 901, EP 100 615, GB-1 255 161 Es wurden neue Cycloalkyl- bzw. Heterocyclyl-carbonsäureanilide der allgemeinen Formel (I)

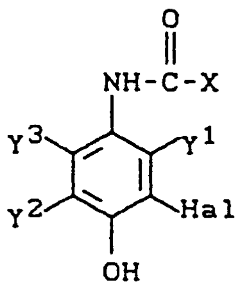

(I)

in welcher

| | |
|---|---|
| X | für gegebenenfalls Alkyl-substituiertes Cyclobutyl, Cyclopentyl, Cyclohexyl, Cycloheptyl, 1-Alkyl substituiertes Cyclopropyl oder einen gegebenenfalls Alkyl-substituierten Heterocyclus steht, |
| Hal | für Halogen steht und |
| $Y^1$, $Y^2$ und $Y^3$ | unabhängig voneinander für Wasserstoff, Halogen, gegebenenfalls Halogen-substituiertes Alkyl, gegebenenfalls Halogen-substituiertes Alkoxy oder gegebenenfalls Halogen-substituiertes Alkylthio stehen, |

gefunden.

Die substituierten Cycloalkyl- bzw. Heterocyclyl-carbonsäureanilide der Formel (I) enthalten ein oder mehrere Asymmetriezentren und können somit in Form von Diastereomeren oder Diastereomerengemischen vorliegen, die in unterschiedlichen Mengenverhältnissen anfallen. Vorwiegend fallen sie als Racemate an.

Weiterhin wurde gefunden, daß man die neuen substituierten Cycloalkyl- bzw. Heterocyclyl-carbonsäureanilide der Formel (I)

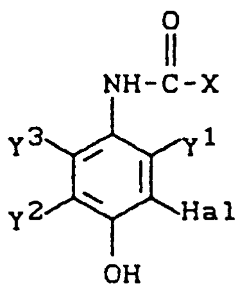

(I)

in welcher

| | |
|---|---|
| X | für gegebenenfalls Alkyl-substituiertes Cyclobutyl, Cyclopentyl, Cyclohexyl, Cycloheptyl, 1-Alkyl substituiertes Cyclopropyl oder einen gegebenenfalls Alkyl-substituierten Heterocyclus, |
| Hal | für Halogen und |
| $Y^1$, $Y^2$ und $Y^3$ | gleich oder verschieden sind und für Wasserstoff, Halogen, gegebenenfalls Halogen-substituiertes Alkyl, gegebenenfalls Halogen-substituiertes Alkoxy oder gegebenenfalls Halogen-substituiertes Alkylthio stehen, |

erhält, wenn man Aminophenole der Formel (II)

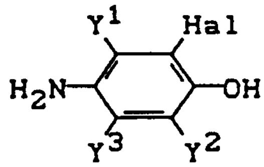

(II)

in welcher

Hal, $Y^1$, $Y^2$ und $Y^3$ die oben angegebenen Bedeutungen haben,

mit Carbonsäure-Derivaten der Formel (III)

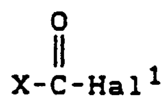

(III)

in welcher

X die oben angegebene Bedeutung hat und

$Hal^1$ für Halogen, vorzugsweise Chlor, oder eine bei Acylierungsreaktionen gebräuchliche Abgangsgruppe, vorzugsweise einen aktivierenden Esterrest, steht

gegebenenfalls in Gegenwart eines Säureakzeptors und gegebenenfalls in Gegenwart eines Lösungs- oder Verdünnungsmittels umsetzt.

Schließlich wurde gefunden, daß die substituierten Cycloalkyl- bzw. Heterocyclyl-carbonsäureanilide der Formel (I) u. a. eine hohe fungizide Wirksamkeit besitzen. Die neuen Verbindungen können auch mit anderen bekannten, hochwirksamen Verbindungen in synergistischen Mischungen verwendet werden.

Im Rahmen der vorliegenden Erfindung können die Substituenten im allgemeinen die folgenden Bedeutungen haben.

Halogen kann, überall wo nicht anders angegeben, Fluor, Chlor, Brom und Iod, bevorzugt Fluor, Chlor und Brom bedeuten.

Alkyl, Alkoxy und Alkylthio stehen für einen Rest, der je Alkyleinheit 1 - 6, bevorzugt 1 - 4 und besonders bevorzugt 1 - 3 Kohlenstoffatome hat, z. B. Methyl, Ethyl, n- und iso-Propyl, n-, sec-, iso- und tert.-Butyl, Pentyl, n-Hexyl oder iso-Hexyl, Methoxy, Ethoxy, n- und iso-Propoxy, n-, sec.-, iso- und tert.-Butoxy, Pentoxy und Hexoxy, Methylthio, Ethylthio, n- und iso-Propylthio, n-, sec.-, iso- und tert.-Butylthio, Pentylthio und Hexylthio.

Halogenalkoxy bzw. Halogenalkylthio steht im allgemeinen für einen über Sauerstoff bzw. Schwefel gebundenen, geradkettigen oder verzweigten Kohlenwasserstoffrest mit 1 - 6 Kohlenstoffatomen und 1 - 9 gleichen oder verschiedenen Halogenatomen. Bevorzugt sind Reste mit 1 - 4 Kohlenstoffatomen und 1 - 5 gleichen oder verschiedenen Halogenatomen. Ganz besonders bevorzugt sind Reste mit 1 oder 2 Kohlenstoffatomen und 1 - 3 gleichen oder verschiedenen Halogenatomen. Beispielhaft seien genannt: Trifluormethoxy, Trichlormethoxy, Difluorchlormethoxy, Dichlorfluormethoxy, Difluorethoxy, Trifluorethoxy, Tetrafluorethoxy, Pentafluorethoxy, Trifluormethylthio, Trichlormethylthio, Difluorchlormethylthio, Dichlorfluormethylthio, Difluorethylthio, Trifluormethylthio, Tetrafluorethylthio.

Halogenalkyl hat die Bedeutung von Halogenalkoxy.

Cycloalkyl steht im allgemeinen für einen cyclischen Kohlenwasserstoffrest mit 3 - 10 Kohlenstoffatomen. Bevorzugt sind Reste mit 3 - 7 Kohlenstoffatomen. Beispielhaft seien genannt: Cyclopropyl, Cyclobutyl, Cyclopentyl, Cyclohexyl, Cycloheptyl und Cyclodecanyl.

Die Cycloalkylreste können ein- bis mehrfach substituiert sein. Als Substituenten seien Alkyl mit 1 - 6 Kohlenstoffatomen genannt.

Alkyl hat die bevorzugte und besonders bevorzugte Bedeutung, die bereits weiter oben gegeben wurde.

Heterocyclus kann für einen Rest mit 4 - 7 Ringgliedern, bevorzugt mit 4 - 6 Ringgliedern, stehen, wobei außer Kohlenstoff ein oder mehrere Heteroatome wie Sauerstoff, Schwefel oder Stickstoff, enthalten sind. Bevorzugt sind 5- oder 6-Ringe mit einem oder zwei der genannten Heteroatome, vor allem Sauerstoff. Beispielsweise seien genannt: Oxetanyl, Oxolanyl, Oxanyl, Dioxolanyl und Dioxanyl. Die Heterocyclen können ein- bis mehrfach, gleich oder verschieden substituiert sein durch Alkyl mit 1 - 6, vorzugsweise 1 - 4 und besonders bevorzugt mit 1 oder 2 Kohlenstoffatomen. Beispielsweise seien genannt: 3-Methyl-oxetan-3-yl, 2-Methyl-oxolan-2-yl, 2-Methyl-oxan-2-yl, 5-Methyl-1,3-dioxolan-5-yl, 2-Ethyl-oxolan-2-yl, 2-Ethyl-oxan-2-yl, 5-Ethyl-1,3-dioxolan-5-yl.

Die erfindungsgemäßen substituierten Cycloalkyl- bzw. Heterocyclyl-carbonsäureanilide sind durch die Formel (I) allgemein definiert. Bevorzugt sind Verbindungen der Formel (I), worin

X für gegebenenfalls ein- bis vierfach, gleich oder verschieden durch geradkettiges oder verzweigtes Alkyl mit 1 - 4 Kohlenstoffatomen substituiertes Cyclobutyl, Cyclopentyl, Cyclohexyl oder Cycloheptyl steht $C_1$-$C_4$-Alkyl substituiertes Cyclopropyl steht oder für oder für einen Heterocyclus mit 4 - 6 Ringgliedern steht, wobei außer Kohlenstoff ein oder zwei gleiche oder verschiedene Heteroatome wie Sauerstoff oder Stickstoff enthalten sind. Der Heterocyclus kann ein- bis sechsfach, gleich oder verschieden durch geradkettiges oder verzweigtes Alkyl mit 1 - 4 Kohlenstoffatomen substituiert sein,

Hal für Fluor, Chlor oder Brom steht,

$Y^1$, $Y^2$ und $Y^3$ gleich oder verschieden sind und für Wasserstoff, Fluor, Chlor, Brom, geradkettiges oder verzweigtes Alkyl mit 1 - 4 Kohlenstoffatomen, geradkettiges oder verzweigtes Alkoxy oder Alkylthio mit je 1 - 4 Kohlenstoffatomen, für Halogenalkyl, Halogenalkoxy oder Halogenalkylthio mit je 1 - 4 Kohlenstoffatomen im geradkettigen oder verzweigten Alkylteil und mit 1 - 5 gleichen oder verschiedenen Halogenatomen steht.

Besonders bevorzugt sind Verbindungen der Formel (I), worin

X für ein- oder zweifach, gleich oder verschieden durch geradkettiges oder verzweigtes Alkyl mit 1 - 3 Kohlenstoffatomen substituiertes Cyclopropyl, Cyclobutyl, Cyclopentyl, Cyclohexyl oder Cycloheptyl, für gegebenenfalls ein- oder zweifach, gleich oder verschieden durch geradkettiges oder verzweigtes Alkyl mit 1 - 3 Kohlenstoffatomen substituiertes Oxanyl, Oxolanyl, Dioxanyl, Dioxolanyl oder Oxetanyl steht,

$Y^1$, $Y^2$ und $Y^3$ gleich oder verschieden sind und für Wasserstoff, Fluor, Chlor, Brom, Methyl oder Trifluormethyl stehen und

Hal für Fluor, Chlor oder Brom steht.

Ganz besonders bevorzugt sind Verbindungen der Formel (I), worin

X für in 1-Stellung durch Methyl oder Ethyl substituiertes Cyclopropyl, Cyclobutyl, Cyclopentyl, Cyclohexyl oder Cycloheptyl steht, welche durch einen weiteren Alkylrest mit 1 - 3 Kohlenstoffatomen zusätzlich substituiert sind,

Hal für Fluor, Chlor oder Brom steht und

$Y^1$, $Y^2$ und $Y^3$ gleich oder verschieden sind und für Wasserstoff, Fluor, Chlor, Brom oder Trifluormethyl stehen.

Verwendet man beispielsweise 2,6-Dichlor-4-amino-phenol und 1-Methyl-1-chlorcarbonylcyclohexan als Ausgangsstoffe, so kann der Reaktionsverlauf durch das folgende Formelschema wiedergegeben werden:

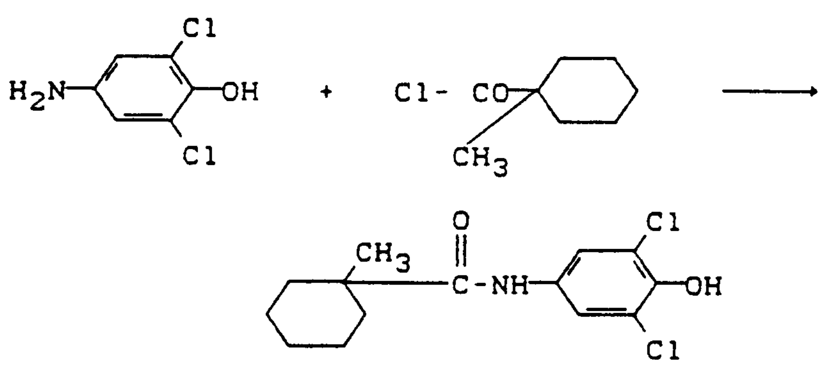

Die zur Durchführung des erfindungsgemäßen Verfahrens als Ausgangsstoffe benötigten Aminophenole sind durch die Formel (II) allgemein definiert. In dieser Formel (II) haben die Reste Hal und $Y^1$ - $Y^3$ die bereits im Zusammenhang mit der Beschreibung der erfindungsgemäßen Verbindungen der Formel (I) gegebenen Bedeutungen. Die Verbindungen sind größtenteils bekannt und nach Analogieverfahren herstellbar (vergl. "Methoden der organischen Chemie", Houben-Weyl, Band VI/1c, Phenole, Teil 1, Georg Thieme Verlag, Stuttgart, 1976, und "Reaktionen der organischen Synthese", Cesare Ferri, S. 81, 89, 91, 97, 118, 120, 122, 124, 126, 128, Georg Thieme Verlag, Stuttgart, 1978).

Die 4-Amino-2-chlor- bzw. -2-brom-6-trifluormethylphenole sind bekannt aus Jp. Kokai Tokkyo Koho Jp 61/126055 und z. B. 4-Amino-2,3,5,6-tetrafluorphenol aus Zh. Org. Khim. 10(9), 1923-1927 (1974). Die Verbindungen der Formel (II A)

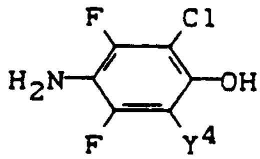

(II A)

in welcher

$Y^4$ für Fluor oder Chlor steht, sind noch neu und Gegenstand einer eigenen, noch nicht vorveröffentlichten deutschen Anmeldung P 3 804 288 und z. B. hergestellt werden aus entsprechenden Hydroxybenzoesäuren der Formel (VA)

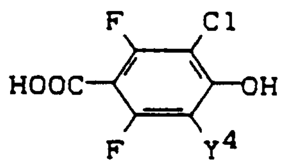

(VA)

durch Decarboxylierung mit anschließender Nitrierung der entstehenden Phenole der Formeln (VI A)

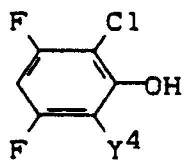

(VI A)

zu den Nitroverbindungen der Formel (VII A)

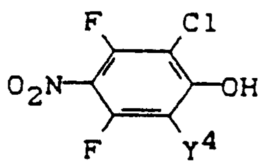

(VII A)

die dann hydriert werden, z. B. mit Wasserstoff und Raney-Nickel, zu den entsprechenden Aminen der Formeln (II A).

Auch die Verbindungen der Formeln (VII A) sind noch neu und Gegenstand der obigen, nicht vorveröffentlichten deutschen Anmeldung.

Die zur Durchführung des erfindungsgemäßen Verfahrens außerdem benötigten Cycloalkancarbonsäure-Derivate sind durch die Formel (III), in der X für Cycloalkyl steht, allgemein definiert. In dieser Formel (III) haben die Reste X und $Hal^1$ die bereits im Zusammenhang mit der Beschreibung der erfindungsgemäßen Verbindungen der Formel (I) gegebenen Bedeutungen. Die Verbindungen sind bekannt und nach Analogieverfahren herstellbar (vergl. Diversi et. al., Synthesis 1971, 258; US 3 674 831; "Reaktionen der organischen Synthese" Cesare Ferri, S. 460, 461, 1978, Georg Thieme Verlag, Stuttgart). Die zur Durchführung des erfindungsgemäßen Verfahrens außerdem benötigten heterocyclischen Carbonsäure-Derivate sind ebenfalls durch die Formel (III), in der X für Heterocyclyl steht, definiert. Die Verbindungen sind bekannt (vergl. DE 1 900 202; DE 2 212 641) und können nach Analogie-Verfahren hergestellt werden.

Das erfindungsgemäße Verfahren wird gegebenenfalls in Gegenwart von Säureakzeptoren durchgeführt. Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -ethylat, ferner aliphatische, aromatische und heterocyclische Amine, beispielsweise Triethylamin, Trimethylamin, Dimethylanilin, 1,8-Diazabicyclo(5,4,0)-undec-7-en, Dimethylbenzylamin und Pyridin.

Zur Durchführung des erfindungsgemäßen Verfahrens setzt man vorzugsweise auf ein Mol Aminophenol der allgemeinen Formel (II) 1 - 2 Mol, insbesondere 1 - 1,4 Mol, der Verbindungen der allgemeinen Formel (III) ein.

Als Verdünnungsmittel zur Durchführung des erfindungsgemäßen Verfahrens kommen praktisch alle inerten organischen Verdünnungsmittel in Frage. Hierzu gehören insbesondere aliphatische und aromatische, gegebenenfalls halogenierte Kohlenwasserstoffe wie Pentan, Hexan, Heptan, Cyclohexan, Petrolether, Benzin, Ligroin, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methylethyl-, Methylisopropyl- und Methylisobutylketon, Ester wie Essigsäuremethylester und - ethylester, Nitrile wie z. B. Acetonitril und Propionitril, Amide wie z. B. Dimethylformamid, Dimethylacetamid und N-Methylpyrrolidon sowie Dimethylsulfoxid, Tetramethylensulfon und Hexamethylphosphorsäuretriamid.

Das erfindungsgemäße Verfahren wird im allgemeinen bei Temperaturen zwischen -50 °C und 120 °C durchgeführt. Bevorzugt wird der Bereich zwischen 0 °C und 110 °C. Die Umsetzungen werden im allgemeinen bei Normaldruck durchgeführt.

Die Aufarbeitung erfolgt nach üblichen Methoden, beispielsweise durch Extraktion der Produkte mit Toluol oder Methylenchlorid aus der mit Wasser verdünnten Reaktionsmischung, Waschen der organischen Phase mit Wasser, Trocknen und Destillieren oder sogenanntes "Andestillieren:, d. h. längeres Erhitzen unter vermindertem Druck auf mäßig erhöhte Temperaturen, um sie von den letzten flüchtigen Bestandteilen zu befreien, oder durch chromatographische Reinigung über Kieselgel oder z. B. durch Kristallisation. Zur Charakterisierung der Verbindungen dienen Brechungsindex, Schmelzpunkt, $R_f$-Wert oder Siedepunkt.

Die erfindungsgemäßen Wirkstoffe sind für den Gebrauch zur Bekämpfung von Schädlingen, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenvertraglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut,

und des Bodens.

Die Wirkstoffe Kannen in die üblichen Formulierungen übergeführt werden, wie Losungen, Emulsionen, Suspensionen, Pulver, Schaume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Losungsmitteln, unter Druck stehenden verflüssigten Gasen und/oder festen Trägerstoffen, gegebenenfalls unter Verwendung von oberflachenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Herstellungsbeispiele

Beispiel 1:

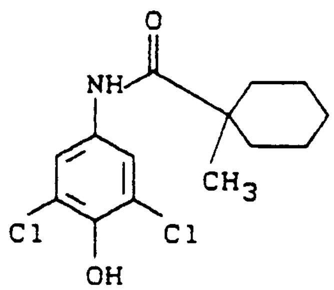

18,5 g (0,085 Mol) 4-Amino-2,6-dichlorphenol werden in 150 ml Tetrahydrofuran gelöst und zunächst mit 8,6 g (0,085 Mol) Triethylamin, dann bei 0 °C Innentemperatur mit 15 g (0,094 Mol) 1-Methylcyclohexancarbonsäurechlorid versetzt. Man rührt über Nacht bei 20 °C und fügt dann zur Vervollständigung der Reaktion erneut 5 g Carbonsäurechlorid und 2,8 g Triethylamin zur Reaktionsmischung. Nach 2 Stunden wird auf Eis gegossen und der abgesaugte Feststoff aus Toluol umkristallisiert. Man erhält die obengenannte Verbindung mit dem Schmelzpunkt 140 °C; Ausbeute: 22,3 g (= 87 % der Theorie).

Analog werden die Verbindungen der Formel (I) erhalten:

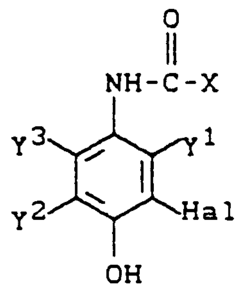

(I)

| Beisp. Nr. | X | Hal | $Y^1$ | $Y^2$ | $Y^3$ | physikalische Daten |
|---|---|---|---|---|---|---|
| 2 | 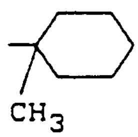 | Cl | H | H | H | |
| 3 | " | Cl | Cl | H | H | Fp. 141° C |
| 4 | " | Cl | F | F | F | Fp. 138° C |
| 5 | 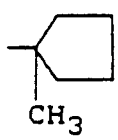 | Cl | H | Cl | H | |
| 6 | 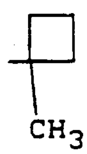 | Cl | H | Cl | H | |
| 7 | 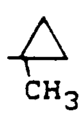 | Cl | H | Cl | H | Fp. 167° C |
| 8 | 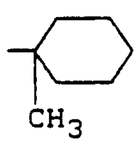 | Cl | H | Cl | H | |

| Beisp. Nr. | X | Hal | $Y^1$ | $Y^2$ | $Y^3$ | physikalische Daten |
|---|---|---|---|---|---|---|
| 9 | 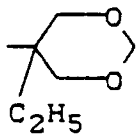 | Cl | H | Cl | H | |
| 10 | 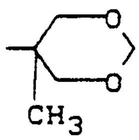 | Cl | H | Cl | H | |
| 11 | 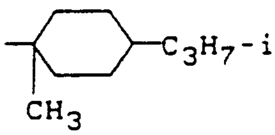 | Cl | H | Cl | H | Fp. 77-81° C |
| 12 | 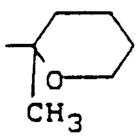 | Cl | H | Cl | H | |
| 13 | 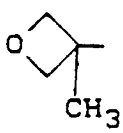 | Cl | H | Cl | H | |
| 14 | 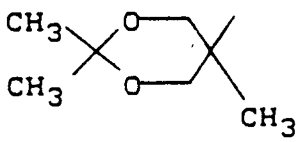 | Cl | H | Cl | H | |

| Beisp. Nr. | X | Hal | $Y^1$ | $Y^2$ | $Y^3$ | physikalische Daten |
|---|---|---|---|---|---|---|
| 15 | 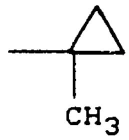 | Cl | Cl | H | H | |
| 16 | 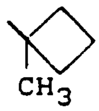 | Cl | Cl | H | H | |
| 17 | 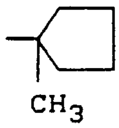 | Cl | Cl | H | H | |
| 18 | 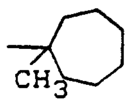 | Cl | Cl | H | H | |
| 19 | 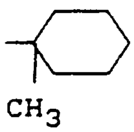 | F | Cl | H | F | |
| 20 | 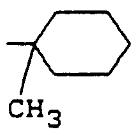 | F | F | Cl | F | |
| 21 | 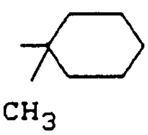 | F | F | H | H | |
| 22 | 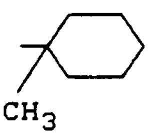 | Br | Br | H | H | |
| 23 | 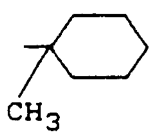 | Cl | F | Cl | F | |
| 24 | 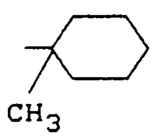 | F | Cl | Cl | F | |

Herstellung der Ausgangsverbindungen

Beispiel A1

3,5-Dichlor-2,6-difluor-4-hydroxybenzoesäure

In einer Rührapparatur werden 300 g Kaliumhydroxid, 600 ml Wasser, 15 g Tetrabutylammoniumchlorid und 135 g 3,5-Dichlor-2,4,6-trifluorbenzotrifluorid vorgelegt und dann für 5 Stunden am Rückfluß erhitzt. Nach Ende der Reaktion wird abgekühlt und durch Zutropfen von Salzsäure sauer gestellt. Das Festprodukt wird abgesaugt und im Vakuum getrocknet. Ausbeute: 93 g mit einem Schmelzpunkt 102 - 105 °C.

Beispiel A2

3-Chlor-2,5,6-trifluor-4-hydroxy-benzoesäure

Analog Beispiel A1 werden aus 400 g NaOH, 1200 ml Wasser, 15 g Tetraethylammoniumchlorid und 276 g 3-Chlortetrafluorbenzotrifluorid bei 6-stündigem Erhitzen unter Rückfluß 238 g Produkt erhalten mit einem Schmelzpunkt von 87 - 90 °C.

Beispiel A3

2,6-Dichlor-3,5-difluorphenol

50 g 3,5-Dichlor-2,6-difluor-4-hydroxy-benzoesäure und 10 ml Dimethylformamid werden vermischt und erhitzt. Bei 105 - 130 °C entwickelt sich Kohlendioxid und man läßt bei dieser Temperatur ausreagieren. Anschließend rührt man 200 ml Toluol und danach 80 ml Wasser ein, trennt die Phasen, trocknet die organische Phase und destilliert anschließend. Man erhält 34 g des Produktes mit einem Siedepunkt von 87 - 88 °C und einem Brechungsindex von $n_D^{20}$ : 1,5310.

Beispiel A4

Analog Beispiel A3 erhält man 2-Chlor-3,5,6-trifluorphenol, mit einem Siedepunkt 68 - 70 °C / 20 mbar.

Beispiel A5

2,6-Dichlor-3,5-difluor-4-nitro-phenol

In 70 ml Essigsäure werden 20 g 2,6-Dichlor-3,5-difluorphenol vorgelegt und 8 g 98 %ige Salpetersäure zugetropft. Anschließend wird für 2 Stunden bei Raumtemperatur nachgerührt, in 150 ml Dichlormethan aufgenommen und zweimal mit Wasser gewaschen. Nach Abdestillieren des Dichlormethans verbleiben 18 g Produkt. Nach GC-Analyse 94 %ig.

Beispiel A6

2-Chlor-3,5,6-trifluor-4-nitrophenol

Analog Beispiel A5 werden durch Nitrierung aus 28 g 2-Chlor-3,5,6-trifluorphenol 25 g 2-Chlor-3,5,6-trifluor-4-nitrophenol erhalten mit einer Reinheit von 93 % und einem Schmelzpunkt von 107 - 109 °C.

Beispiel A7

2,6-Dichlor-3,5-difluor-4-amino-phenol

18 g 2,6-Dichlor-3,5-difluor-4-nitrophenol werden in 100 ml Methanol in Gegenwart von 1,5 g Raney-Nickel bei 25 - 45 °C mit 30 - 50 bar Wasserstoff bis zum Ende der Wasserstoffaufnahme hydriert. Nach Filtration wird die Lösung unter vermindertem Druck vom Lösungsmittel befreit. Es verbleiben 13 g Aminophenol (GC-Reinheit 98,4 %); Fp. 151 °C.

Beispiel A8

2-Chlor-3,5,6-trifluor-4-amino-phenol

Analog Beispiel A7 werden aus 25 g 2-Chlor-3,5,6-trifluor-4-nitro-phenol in 120 ml Methanol und 2 g Raney-Nickel durch Hydrierung 20 g Aminophenol (GC-Reinheit 97 %) erhalten.

Anwendungsbeispiele

Im nachfolgenden Anwendungsbeispiel wird die als Fungizid bekannte Verbindung der Formel A

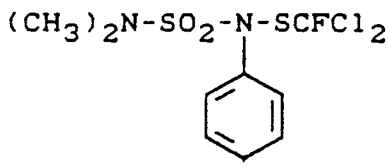

(A)

N,N-Dimethyl-N-phenyl-N'-(fluordichlormethylthio)-sulfamid [K. H. Büchel "Pflanzenschutz und Schädlingsbekämpfung", Georg Thieme Verlag, Stuttgart, S. 141 (1977)] als Vergleichssubstanz verwendet.

Beispiel

Botrytis-Test (Bohne) / protektiv

| | |
|---|---|
| Lösungsmittel: | 4,7 Gewichtsteile Aceton |
| Emulgator: | 0,3 Gewichtsteile Alkyl-aryl-polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20 °C aufgestellt. 3 Tage nach der Inokulation wird die Größe der Befallsflecken auf den Blättern ausgewertet.

Eine deutliche Überlegenheit in der Wirksamkeit gegenüber dem Stand der Technik zeigen in diesem Test z. B. die Verbindungen gemäß den Herstellungsbeispielen: 1 und 3.

**Patentansprüche**
**Patentansprüche für folgende Vertragsstaaten : BE, CH, DE, FR, GB, IT, LI, NL**

**1.** Substituierte Cycloalkyl- bzw. Heterocyclyl-carbonsäureanilide der allgemeinen Formel (I)

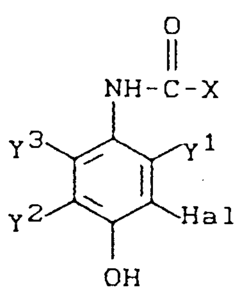

(I)

in welcher

X für gegebenenfalls Alkyl-substituiertes Cyclobutyl, Cyclopentyl, Cyclohexyl, Cycloheptyl, 1-Alkyl substituiertes Cyclopropyl oder einen gegebenenfalls Alkylsubstituierten Heterocyclus steht,

Hal für Halogen steht und

$Y^1$, $Y^2$ und $Y^3$ unabhängig voneinander für Wasserstoff, Halogen, gegebenenfalls Halogen-substituiertes Alkyl, gegebenenfalls Halogen-substituiertes Alkoxy oder für gegebenenfalls Halogen-substituiertes Alkylthio stehen.

**2.** Substituierte Cycloalkyl- bzw. Heterocyclyl-carbonsäureanilide gemäß Anspruch 1, wobei in der allgemeinen Formel (I)

X für gegebenenfalls jeweils ein- bis vierfach, gleich oder verschieden durch geradkettiges oder verzweigtes Alkyl mit 1 bis 4 Kohlenstoffatomen substituiertes Cyclobutyl, Cyclopentyl, Cyclohexyl oder Cycloheptyl steht, ferner für $C_1$-$C_4$-Alkyl substituiertes Cyclopropyl steht oder für einen gegebenenfalls ein- bis sechsfach, gleich oder verschieden durch geradkettiges oder verzweigtes Alkyl mit 1 bis 4 Kohlenstoffatomen substituierten Heterocyclus mit 4 bis 6 Ringgliedern steht, wobei die Ringglieder Kohlenstoffatome und ein oder zwei, gleiche oder verschiedene Sauerstoff- oder Stickstoffatome sind,

Hal für Fluor, Chlor oder Brom steht und

$Y^1$, $Y^2$ und $Y^3$ unabhängig voneinander für Wasserstoff, Fluor, Chlor, Brom, geradkettiges oder verzweigtes Alkyl mit 1 bis 4 Kohlenstoffatomen, für geradkettiges oder versweigtes Alkoxy oder Alkylthio mit jeweils 1 bis 4 Kohlenstoffatomen je Alkylteil, für Halogenalkyl, Halogenalkoxy oder Halogenalkylthio mit je 1 - 4 Kohlenstoffatomen je geradkettigen oder verzweigten Alkylteil und 1 - 5 gleichen oder verschiedenen Halogenatomen stehen.

**3.** Substituierte Cycloalkyl- bzw. Heterocyclylcarbonsäureanilide gemäß Anspruch 1, wobei in der allgemeinen Formel (I)

X für ein- oder zweifach, gleich oder verschieden jeweils durch geradkettiges oder verzweigtes Alkyl mit 1 - 3 Kohlenstoffatomen substituiertes Cyclopropyl, Cyclobutyl, Cyclopentyl, Cyclohexyl oder Cycloheptyl steht, ferner für gegebenenfalls ein- oder zweifach, gleich oder verschieden durch geradkettiges oder verzweigtes Alkyl mit 1 - 3 Kohlenstoffatomen jeweils substituiertes Oxetanyl, Oxanyl, Oxolanyl, Dioxanyl oder Dioxolanyl steht,

$Y^1$, $Y^2$ und $Y^3$ unabhängig voneinander für Wasserstoff, Fluor, Chlor, Brom, Methyl oder Trifluormethyl stehen und

Hal für Fluor, Chlor oder Brom steht.

**4.** Substituierte Cycloalkyl- bzw. Heterocyclylcarbonsäureanilide gemäß Anspruch 1, wobei in der Formel (I)

X für in 1-Stellung durch Methyl oder Ethyl substituiertes Cyclopropyl, Cyclobutyl, Cyclopentyl, Cyclohexyl oder Cycloheptyl steht, wobei jeder Rest durch einen weiteren geradkettigen oder verzweigten Alkylrest mit 1 - 3 Kohlenstoffatomen substituiert sein kann,

Hal für Fluor, Chlor oder Brom steht und

$Y^1$, $Y^2$ und $Y^3$ unabhängig voneinander für Wasserstoff, Fluor, Chlor, Brom oder Trifluormethyl stehen.

**5.** Verfahren zur Herstellung von Cycloalkyl- bzw. Heterocyclyl-carbonsäureaniliden der allgemeinen Formel (I)

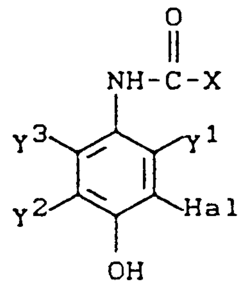

(I)

in welcher

X, Hal, $Y^1$, $Y^2$ und $Y^3$ die in Anspruch 1 angegebenen Bedeutungen haben

dadurch gekennzeichnet, daß man Aminophenole der allgemeinen Formel (II)

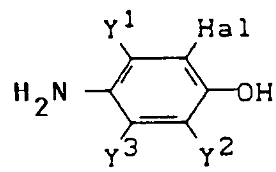

(II)

mit Carbonsäure-Derivaten der Formel (III)

X-CO-$Hal^1$ (III)

in welchen

X, $Y^1$, $Y^2$, $Y^3$ und Hal die oben angegebenen Bedeutungen haben und

$Hal^1$ für Halogen oder eine gebräuchliche Abgangsgruppe steht,

gegebenenfalls in Gegenwart eines Säureakzeptors und gegebenenfalls in Gegenwart eines Lösungs- oder Verdünnungsmittels umsetzt.

**6.** Schädlingsbekämpfungsmittel, gekennzeichnet durch einen Gehalt an mindestens einem substituierten Cycloalkyl- bzw. Heterocyclyl-carbonsäureanilid der Formel (I) nach den Ansprüchen 1 oder 5.

**7.** Verwendung von substituierten Cycloalkyl- bzw. Heterocyclyl-carbonsäureaniliden der Formel (I) nach den Ansprüchen 1 oder 5 zur Bekämpfung von Schädlingen.

**8.** Verfahren zur Bekämpfung von Schädlingen, dadurch gekennzeichnet, daß man substituierte Cycloalkyl- bzw. Heterocyclyl-carbonsäureanilide der Formel (I) nach den Ansprüchen 1 oder 5 auf Schädlinge und/oder ihren Lebensraum einwirken läßt.

**9.** Verfahren zur Herstellung von Schädlingsbekämpfungsmitteln, dadurch gekennzeichnet, daß man substituierte Cycloalkyl- bzw. Heterocyclyl-carbonsäureanilide der Formel (I) nach den Ansprüchen 1 oder 5 mit Streckmitteln und/oder oberflächenaktiven Mitteln vermischt.

**Patentansprüche für folgenden Vertragsstaat : ES**

**1.** Verfahren zur Herstellung von Cycloalkyl- bzw. Heterocyclyl-carbonsäureaniliden der allgemeinen Formel (I)

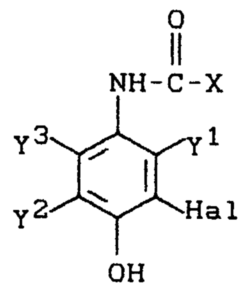

(I)

in welcher

X für gegebenenfalls Alkyl-substituiertes Cyclobutyl, Cyclopentyl, Cyclohexyl, Cycloheptyl, 1-Alkyl substituiertes Cyclopropyl oder einen gegebenenfalls Alkyl-substituierten Heterocyclus steht,

Hal für Halogen steht und

$Y^1$, $Y^2$ und $Y^3$ unabhängig voneinander für Wasserstoff, Halogen, gegebenenfalls Halogen-substituiertes Alkyl, gegebenenfalls Halogen-substituiertes Alkoxy oder für gegebenenfalls Halogen-substituiertes Alkylthio stehen,

dadurch gekennzeichnet, daß man Aminophenole der allgemeinen Formel (II)

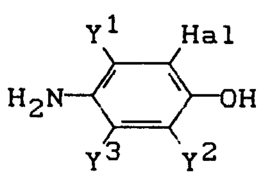

(II)

mit Carbonsäure-Derivaten der Formel (III)

$X\text{-}CO\text{-}Hal^1$ (III)

in welchen

X, $Y^1$, $Y^2$, $Y^3$ und Hal die oben angegebenen Bedeutungen haben und

$Hal^1$ für Halogen oder eine gebräuchliche Abgangsgruppe steht,

gegebenenfalls in Gegenwart eines Säureakzeptors und gegebenenfalls in Gegenwart eines Lösungs- oder Verdünnungsmittels umsetzt.

**2.** Verfahren gemäß Anspruch 1, wobei in der allgemeinen Formel (I)

X für gegebenenfalls jeweils ein- bis vierfach, gleich oder verschieden durch geradkettiges oder verzweigtes Alkyl mit 1 bis 4 Kohlenstoffatomen substituiertes Cyclobutyl, Cyclopentyl, Cyclohexyl oder Cycloheptyl steht, ferner für $C_1$-$C_4$-Alkyl substituiertes Cyclopropyl steht oder für einen gegebenenfalls ein-bis sechsfach, gleich oder verschieden durch geradkettiges oder verzweigtes Alkyl mit 1 bis 4 Kohlenstoffatomen substituierten Heterocyclus mit 4 bis 6 Ringgliedern steht, wobei die Ringglieder Kohlenstoffatome und ein oder zwei, gleiche oder verschiedene Sauerstoff- oder Stickstoffatome sind,

Hal für Fluor, Chlor oder Brom steht und

$Y^1$, $Y^2$ und $Y^3$ unabhängig voneinander für Wasserstoff, Fluor, Chlor, Brom, geradkettiges oder verzweigtes Alkyl mit 1 bis 4 Kohlenstoffatomen, für geradkettiges oder verzweigtes Alkoxy oder Alkylthio mit jeweils 1 bis 4 Kohlenstoffatomen je Alkylteil, für Halogenalkyl, Halogenalkoxy oder Halogenalkylthio mit je 1 - 4 Kohlenstoffatomen je geradkettigen oder verzweigten Alkylteil und 1 - 5 gleichen oder verschiedenen Halogenatomen stehen.

**3.** Verfahren gemäß Anspruch 1, wobei in der allgemeinen Formel (I)

X für ein- oder zweifach, gleich oder verschieden jeweils durch geradkettiges oder verzweigtes Alkyl mit 1 - 3 Kohlenstoffatomen substituiertes Cyclopropyl, Cyclobutyl, Cyclopentyl, Cyclohexyl oder Cycloheptyl steht, ferner für gegebenenfalls ein- oder zweifach, gleich oder verschieden durch geradkettiges oder verzweigtes Alkyl mit 1 - 3 Kohlenstoffatomen jeweils substituiertes Oxetanyl, Oxanyl, Oxolanyl, Dioxanyl oder Dioxolanyl steht,

$Y^1$, $Y^2$ und $Y^3$ unabhängig voneinander für Wasserstoff, Fluor, Chlor, Brom, Methyl oder Trifluormethyl stehen und

Hal für Fluor, Chlor oder Brom steht.

**4.** Verfahren gemäß Anspruch 1, wobei in der Formel (I)

X für in 1-Stellung durch Methyl oder Ethyl substituiertes Cyclopropyl, Cyclobutyl, Cyclopentyl, Cyclohexyl oder Cycloheptyl steht, wobei jeder Rest durch einen weiteren geradkettigen oder verzweigten Alkylrest mit 1 - 3 Kohlenstoffatomen substituiert sein kann,

Hal für Fluor, Chlor oder Brom steht und

$Y^1$, $Y^2$ und $Y^3$ unabhängig voneinander für Wasserstoff, Fluor, Chlor, Brom oder Trifluormethyl stehen.

**5.** Schädlingsbekämpfungsmittel, gekennzeichnet durch einen Gehalt an mindestens einem substituierten Cycloalkyl- bzw. Heterocyclyl-carbonsäureanilid der Formel (I) nach den Ansprüchen 1 oder 5.

**6.** Verwendung von substituierten Cycloalkyl- bzw. Heterocyclyl-carbonsäureaniliden der Formel (I) nach den Ansprüchen 1 oder 5 zur Bekämpfung von Schädlingen.

**7.** Verfahren zur Bekämpfung von Schädlingen, dadurch gekennzeichnet, daß man substituierte Cycloalkyl- bzw. Heterocyclyl-carbonsäureanilide der Formel (I) nach den Ansprüchen 1 oder 5 auf Schädlinge und/oder ihren Lebensraum einwirken läßt.

**8.** Verfahren zur Herstellung von Schädlingsbekämpfungsmitteln, dadurch gekennzeichnet, daß man substituierte Cycloalkyl- bzw. Heterocyclyl-carbonsäureanilide der Formel (I) nach den Ansprüchen 1 oder 5 mit Streckmitteln und/oder oberflächenaktiven Mitteln vermischt.

**Claims**
**Claims for the following Contracting States : BE, CH, DE, FR, GB, IT, LI, NL**

**1.** Substituted cycloalkyl- or heterocyclyl-carboxanilides of the general formula (I)

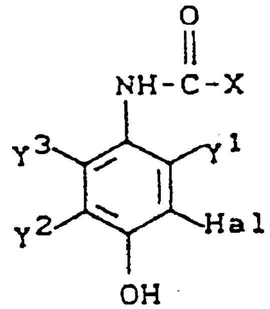

(I)

in which

X represents optionally alkyl-substituted cyclobutyl, cyclopentyl, cyclohexyl, cycloheptyl, 1-alkyl-substituted cyclopropyl or an optionally alkyl-substituted heterocyclic,

Hal represents halogen and

$Y^1$, $Y^2$ and $Y^3$ independently of one another represent hydrogen, halogen, optionally halogen-substituted alkyl, optionally halogen-substituted alkoxy or optionally halogen-substituted alkylthio.

**2.** Substituted cycloalkyl- and heterocyclyl-carboxanilides according to Claim 1, where in the general formula (I)

X represents cyclobutyl, cyclopentyl, cyclohexyl or cycloheptyl, each of which is optionally monosubstituted to tetrasubstituted by identical or different, straight-chain or branched alkyl substituents having 1 to 4 carbon atoms, or represents $C_1$-$C_4$-alkyl-substituted cyclopropyl or represents a heterocycle having 4 to 6 ring members which is optionally monosubstitinted to hexasubstituted by identical or different, straight-chain or branched alkyl substituents having 1 to 4 carbon atoms, the ring members being carbon atoms and one or two identical or different oxygen or nitrogen atoms,

Hal represents fluorine, chlorine or bromine and

$Y^1$, $Y^2$ and $Y^3$ independently of one another represent hydrogen, fluorine, chlorine, bromine, straight-chain or branched alkyl having 1 to 4 carbon atoms, or represent straight-chain or branched alkoxy or alkylthio, each having 1 to 4 carbon atoms per alkyl moiety, or represent halogenoalkyl, halogenoalkoxy or halogenoalkylthio, each having 1-4 carbon atoms in the straight-chain or branched alkyl moiety and having 1-5 identical or different halogen atoms.

**3.** Substituted cycloalkyl- or heterocyclyl-carboxanilides according to Claim 1, where in the general formula (I)

X represents cyclopropyl, cyclobutyl, cyclopentyl, cyclohexyl or cycloheptyl, each of which is monosubstituted or disubstituted by identical or different, straight-chain or branched alkyl substituents having 1-3 carbon atoms, or represents oxetanyl, oxanyl, oxolanyl, dioxanyl or dioxolanyl, each of which is optionally monosubstituted or disubstituted by identical or different, straight-chain or branched alkyl substituents having 1-3 carbon atoms,

$Y^1$, $Y^2$, and $Y^3$ independently of one another represent hydrogen, fluorine, chlorine, bromine, methyl or trifluoromethyl and

Hal represents fluorine, chlorine or bromine.

**4.** Substituted cycloalkyl- and heterocyclyl-carboxanilides according to Claim 1, where in the formula (I)

X represents cyclopropyl, cyclobutyl, cyclopentyl, cyclohexyl or cycloheptyl, each of which is substituted in the 1-position by methyl or ethyl and each of which can be substituted by another straight-chain or branched alkyl radical having 1-3 carbon atoms,

Hal represents fluorine, chlorine or bromine and

$Y^1$, $Y^2$ and $Y^3$ independently of one another represent hydrogen, fluorine, chlorine, bromine or trifluoromethyl.

**5.** Process for the preparation of cycloalkyl- or heterocyclyl-carboxanilides of the general formula (I)

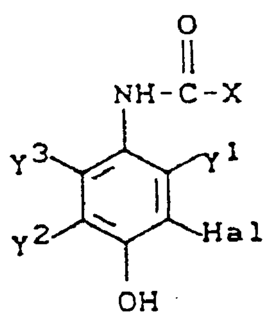

(I)

in which

X, Hal, $Y^1$, $Y^2$ and $Y^3$ have the meanings stated in Claim 1,

characterized in that aminophenols of the general formula (II)

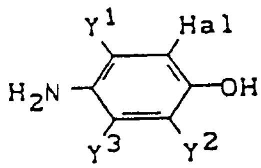

(II)

are reacted with carboxylic acid derivatives of the formula (III)

X-CO-Hal[1] (III)

in which

X, $Y^1$, $Y^2$, $Y^3$ and Hal have the abovementioned meanings and

$Hal^1$ represents halogen or a customary leaving group,

if appropriate in the presence of an acid acceptor and if appropriate in the presence of a solvent or diluent.

**6.** Pesticides, characterized in that they contain at least one substituted cycloalkyl- or heterocyclylcarboxanilide of the formula (I) according to Claims 1 or 5.

**7.** Use of substituted cycloalkyl- or heterocyclylcarboxanilides of the formula (I) according to Claims 1 or 5 for combating pests.

**8.** Method of combating pests, characterized in that substituted cycloalkyl- or heterocyclyl-carboxanilides of the formula (I) according to Claims 1 or 5 are allowed to act on pests and/or their environment.

**9.** Process for the preparation of pesticides, characterized in that substituted cycloalkyl- or heterocyclylcarboxanilides of the formula (I) according to Claims 1 or 5 are mixed with extenders and/or surface-active agents.

**Claims for the following Contracting State : ES**

**1.** Process for the preparation of cycloalkyl or heterocyclyl-carboxanilides of the general formula (I)

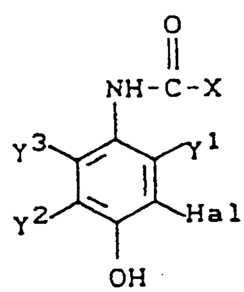

(I)

in which

X represents optionally alkyl-susbtituted cyclobutyl, cyclopentyl, cyclohexyl, cycloheptyl, 1-alkyl-substituted cyclopropyl or an optionally alkyl-substituted heterocycle,

Hal represents halogen and

$Y^1$, $Y^2$ and $Y^3$ independently of one another represent hydrogen, halogen, optionally halogensubstituted alkyl, optionally halogen-substituted alkoxy or optionally halogen-substituted alkylthio,

characterized in that aminophenols of the general formula (II)

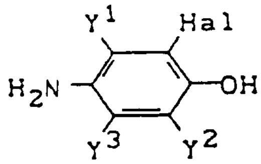

(II)

are reacted with carboxylic acid derivatives of the formula (III)

X-CO-Hal[1] (III)

in which

X, $Y^1$, $Y^2$, $Y^3$ and Hal have the abovementioned meanings and

$Hal^1$ represents halogen or a customary leaving group,

if appropriate in the presence of an acid acceptor and if appropriate in the presence of a solvent or diluent.

**2.** Process according to Claim 1, where in the general formula (I)

X represents cyclobutyl, cyclopentyl, cyclohexyl or cycloheptyl, each of which is optionally monosubstituted to tetrasubstituted by identical or different, straight-chain or branched alkyl substituents having 1 to 4 carbon atoms, or represents $C_1$-$C_4$-alkyl-substituted cyclopropyl or represents a heterocycle having 4 to 6 ring members which is optionally monosubstitinted to hexasubstituted by identical or different, straight-chain or branched alkyl substituents having 1 to 4 carbon atoms, the ring members being carbon atoms and one or two identical or different oxygen or nitrogen atoms,

Hal represents fluorine, chlorine or bromine and

$Y^1$, $Y^2$ and $Y^3$ independently of one another represent hydrogen, fluorine, chlorine, bromine, straight-chain or branched alkyl having 1 to 4 carbon atoms, or represent straight-chain or branched alkoxy or alkylthio, each having 1 to 4 carbon atoms per alkyl moiety, or represent halogenoalkyl, halogenoalkoxy or halogenoalkylthio, each having 1-4 carbon atoms in the straight-chain or branched alkyl moiety and having 1-5 identical or different halogen atoms.

**3.** Process according to Claim 1, where in the general formula (I)

X represents cyclopropyl, cyclobutyl, cyclopentyl, cyclohexyl or cycloheptyl, each of which is monosubstituted or disubstituted by identical or different, straight-chain or branched alkyl substituents having 1-3 carbon atoms, or represents oxetanyl, oxanyl, oxolanyl, dioxanyl or dioxolanyl, each of which is optionally monosubstituted or disubstituted by identical or different, straight-chain or branched alkyl substituents having 1-3 carbon atoms,

$Y^1$, $Y^2$ and $Y^3$ independently of one another represent hydrogen, fluorine, chlorine, bromine, methyl or trifluoromethyl and

Hal represents fluorine, chlorine or bromine.

**4.** Process according to Claim 1, where in the formula (I)

X represents cyclopropyl, cyclobutyl, cyclopentyl, cyclohexyl or cycloheptyl, each of which is substituted in the 1-position by methyl or ethyl and each of which can be substituted by another straight-chain or branched alkyl radical having 1-3 carbon atoms,

Hal represents fluorine, chlorine or bromine and

$Y^1$, $Y^2$ and $Y^3$ independently of one another represent hydrogen, fluorine, chlorine, bromine or trifluoromethyl.

**5.** Pesticides, characterized in that they contain at least one substituted cycloalkyl or heterocyclylcarboxanilide of the formula (I) according to Claims 1 or 4.

6. Use of substituted cycloalkyl- or heterocyclylcarboxanilides of the formula (I) according to Claims 1 or 4 for combating pests.

7. Method of combating pests, characterized in that substituted cycloalkyl- or heterocyclyl-carboxanilides of the formula (I) according to Claims 1 or 4 are allowed to act on pests and/or their environment.

8. Process for the preparation of pesticides, characterized in that substituted cycloalkyl- or heterocyclyl-carboxanilides of the formula (I) according to Claims 1 or 4 are mixed with extenders and/or surface-active agents.

**Revendications**
**Revendications pour les Etats contractants suivants : BE, CH, DE, FR, GB, IT, LI, NL**

1. Anilides d'acides cycloalkyl- ou hétérocyclylcarboxyliques substitués de formule générale (I)

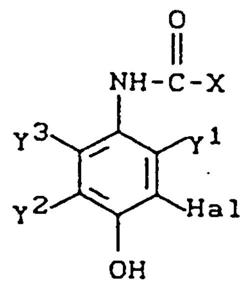

(I)

dans laquelle

| | |
|---|---|
| X | représente un groupe cyclobutyle, cyclopentyle, cyclohexyle, cycloheptyle éventuellement substitué par un radical alkyle, un groupe cyclopropyle portant un substituant 1-alkyle ou un hétérocycle portant éventuellement un substituant alkyle, |
| Hal | représente un halogène et |
| $Y^1$, $Y^2$ et $Y^3$ | représentent, indépendamment les uns des autres, de l'hydrogène, un halogène, un groupe alkyle éventuellement substitué avec un halogène, un groupe alkoxy éventuellement substitué avec un halogène ou un groupe alkylthio éventuellement substitué avec un halogène. |

2. Anilides d'acides cycloalkyl- ou hétérocyclylcarboxyliques substitués suivant la revendication 1, dans la formule générale (I) desquels

| | |
|---|---|
| X | représente un groupe cyclobutyle, cyclopentyle, cyclohexyle ou cycloheptyle portant chacun le cas échéant 1 à 4 substituants, identiques ou différents, alkyle linéaire ou ramifié ayant 1 à 4 atomes de carbone, en outre un groupe cyclopropyle portant un substituant alkyle en $C_1$ à $C_4$ ou un hétérocycle de 4 à 6 chaînons portant le cas échéant 1 à 6 substituants, identiques ou différents, alkyle linéaire ou ramifié ayant 1 à 4 atomes de carbone, les chaînons de l'hétérocycle étant des atomes de carbone et un ou deux atomes d'oxygène ou d'azote, identiques ou différents, |
| Hal | représente du fluor, du chlore, du brome et |
| $Y^1$, $Y^2$ et $Y^3$ | représentent, indépendamment les uns des autres, de l'hydrogène, du fluor, du chlore, du brome, un groupe alkyle linéaire ou ramifié ayant 1 à 4 atomes de carbone, un groupe alkoxy ou alkylthio linéaire ou ramifié ayant chacun 1 à 4 atomes de carbone par partie alkyle, un groupe halogénalkyle, halogénalkoxy ou halogénalkylthio ayant chacun 1 à 4 atomes de carbone par partie alkyle linéaire ou ramifiée et 1 à 5 atomes d'halogènes identiques ou différents. |

3. Anilides d' acides cycloalkyl- ou hétérocyclylcarboxyliques substitués suivant la revendication 1, dans la formule générale (I) desquels

| | |
|---|---|
| X | représente un groupe cyclopropyle, cyclobutyle, cyclopentyle, cyclohexyle ou cycloheptyle portant chacun un ou deux substituants, identiques ou différents, alkyle linéaire ou ramifié ayant 1 à 3 atomes de carbone, en outre un groupe oxétannyle, |

oxannyle, oxolannyle, dioxannyle ou dioxolannyle portant chacun le cas échéant un ou deux substituants, identiques ou différents, alkyle linéaire ou ramifié ayant 1 à 3 atomes de carbone,

$Y^1$, $Y^2$ et $Y^3$ représentent, indépendamment les uns des autres, de l'hydrogène, du fluor, du chlore, du brome, un groupe méthyle ou trifluorométhyle et

Hal représente du fluor, du chlore ou du brome.

**4.** Anilides d'acides cycloalkyl- ou hétérocyclylcarboxyliques substitués suivant la revendication 1, dans la formule (I) desquels

X représente un reste cyclopropyle, cyclobutyle, cyclopentyle, cyclohexyle ou cycloheptyle portant en position 1 un substituant méthyle ou éthyle, chaque reste pouvant être substitué par un autre reste alkyle linéaire ou ramifié ayant 1 à 3 atomes de carbone,

Hal représente du fluor, du chlore ou du brome et

$Y^1$, $Y^2$ et $Y^3$ représentent, indépendamment les uns des autres, de l'hydrogène, du fluor, du chlore, du brome ou un groupe trifluorométhyle.

**5.** Procédé de production d'anilides d'acides cycloalkyl- ou hétérocyclyl-carboxyliques de formule générale (I)

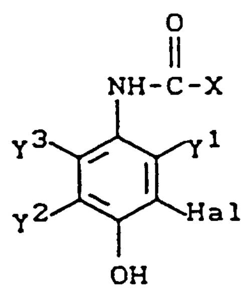

(I)

dans laquelle

X, Hal, $Y^1$, $Y^2$ et $Y^3$ ont les définitions indiquées dans la revendication 1,

caractérisé en ce qu'on fait réagir des aminophénols de formule générale (II)

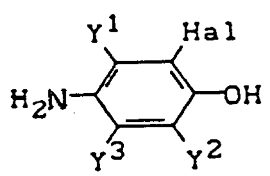

(II)

avec des dérivés d'acides carboxyliques de formule (III)

X-CO-$Hal^1$ (III)

formules dans lesquelles

X, $Y^1$, $Y^2$, $Y^3$ et Hal ont les définitions indiquées ci-dessus et

$Hal^1$ représente un halogène ou un groupe partant usuel,

le cas échéant en présence d'un accepteur d'acide et en la présence éventuelle d'un solvant ou d'un diluant.

**6.** Compositions pesticides, caractérisées par une teneur en au moins un anilide d'acide cycloalkyl- ou hétérocyclyl-carboxylique substitué de formule (I) suivant les revendications 1 ou 5.

**7.** Utilisation d'anilides d'acides cycloalkyl- ou hétérocyclyl-carboxyliques substitués de formule (I) suivant les revendications 1 ou 5 pour combattre des parasites.

**8.** Procédé pour comabattre des parasites, caractérisé en ce qu'on fait agir sur les parasites et/ou sur leur milieu des anilides d'acides cycloalkyl- ou hétérocyclyl-carboxyliques substitués de formule (I) suivant les revendications 1 ou 5.

**9.** Procédé de préparation de compositions pesticides, caractérisé en ce qu'on mélange des anilides d'acides cycloalkyl- ou hétérocyclyl-carboxyliques substitués de formule (I) suivant les revendications 1 ou 5 avec des diluants et/ou des agents tensioactifs.

**Revendications pour l'Etat contractant suivant : ES**

**1.** Procédé de production d'anilides d'acides cycloalkyl- ou hétérocyclyl-carboxyliques de formule générale (I)

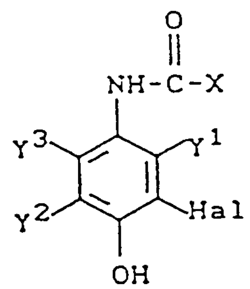

(I)

dans laquelle

| | |
|---|---|
| X | représente un groupe cyclobutyle, cyclopentyle, cyclohexyle, cycloheptyle éventuellement substitué par un radical alkyle, un groupe cyclopropyle portant un substituant 1-alkyle ou un hétérocycle portant éventuellement un substituant alkyle, |
| Hal | représente un halogène et |
| $Y^1$, $Y^2$ et $Y^3$ | représentent, indépendamment les uns des autres, de l'hydrogène, un halogène, un groupe alkyle éventuellement substitué avec un halogène, un groupe alkoxy éventuellement substitué avec un halogène ou un groupe alkylthio éventuellement substitué avec un halogène, |

caractérisé en ce qu'on fait réagir des aminophénols de formule générale (II)

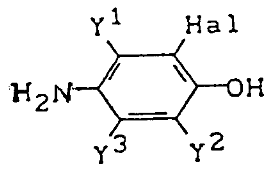

(II)

avec des dérivés d'acides carboxyliques de formule (III)

$X\text{-}CO\text{-}Hal^1$ (III)

formules dans lesquelles

| | |
|---|---|
| X, $Y^1$, $Y^2$, $Y^3$ et Hal | ont les définitions indiquées ci-dessus et |
| $Hal^1$ | représente un halogène ou un groupe partant usuel, |

le cas échéant en présence d'un accepteur d'acide et en la présence éventuelle d'un solvant ou d'un diluant.

**2.** Procédé suivant la revendication 1, dans lequel, dans la formule générale (I)

| | |
|---|---|
| X | représente un groupe cyclobutyle, cyclopentyle, cyclohexyle ou cycloheptyle portant chacun le cas échéant 1 à 4 substituants, identiques ou différents, alkyle linéaire ou ramifié ayant 1 à 4 atomes de carbone, en outre un groupe cyclopropyle portant un substituant alkyle en $C_1$ à $C_4$ ou un hétérocycle de 4 à 6 chaînons portant le cas |

échéant 1 à 6 substituants, identiques ou différents, alkyle linéaire ou ramifié ayant 1 à 4 atomes de carbone, les chaînons de l'hétérocycle étant des atomes de carbone et un ou deux atomes d'oxygène ou d'azote, identiques ou différents,

Hal représente du fluor, du chlore ou du brome et

$Y^1$, $Y^2$ et $Y^3$ représentent, indépendamment les uns des autres, de l'hydrogène, du fluor, du chlore, du brome, un groupe alkyle linéaire ou ramifié ayant 1 à 4 atomes de carbone, un groupe alkoxy ou alkylthio linéaire ou ramifié ayant chacun 1 à 4 atomes de carbone par partie alkyle, un groupe halogénalkyle, halogénalkoxy ou halogénalkylthio ayant chacun 1 à 4 atomes de carbone par partie alkyle linéaire ou ramifiée et 1 à 5 atomes d'halogènes identiques ou différents.

**3.** Procédé suivant la revendication 1, dans lequel, dans la formule générale (I),

X représente un groupe cyclopropyle, cyclobutyle, cyclopentyle, cyclohexyle ou cycloheptyle portant chacun un ou deux substituants, identiques ou différents, alkyle linéaire ou ramifié ayant 1 à 3 atomes de carbone, en outre un groupe oxétannyle, oxannyle, oxolannyle, dioxannyle ou dioxolannyle portant chacun le cas échéant un ou deux substituants, identiques ou différents, alkyle linéaire ou ramifié ayant 1 à 3 atomes de carbone,

$Y^1$, $Y^2$ et $Y^3$ représentent, indépendamment les uns des autres, de l'hydrogène, du fluor, du chlore, du brome, un groupe méthyle ou trifluorométhyle et

Hal représente du fluor, du chlore ou du brome.

**4.** Procédé suivant la revendication 1, dans lequel, dans la formule (I),

X représente un reste cyclopropyle, cyclobutyle, cyclopentyle, cyclohexyle ou cycloheptyle portant en position 1 un substituant méthyle ou éthyle, chaque reste pouvant être substitué par un autre reste alkyle linéaire ou ramifié ayant 1 à 3 atomes de carbone,

Hal représente du fluor, du chlore ou du brome et

$Y^1$, $Y^2$ et $Y^3$ représentent, indépendamment les uns des autres, de l'hydrogène, du fluor, du chlore, du brome ou un groupe trifluorométhyle.

**5.** Compositions pesticides, caractérisées par une teneur en au moins un anilide d'acide cycloalkyl- ou hétérocyclyl-carboxylique substitué de formule (I) suivant les revendications 1 ou 5.

**6.** Utilisation d'anilides d'acides cycloalkyl- ou hétérocyclyl-carboxyliques substitués de formule (I) suivant les revendications 1 ou 5 pour combattre des parasites.

**7.** Procédé pour combattre des parasites, caractérisé en ce qu'on fait agir sur les parasites et/ou sur leur milieu des anilides d'acides cycloalkyl- ou hétérocyclylcarboxyliques substitués de formule (I) suivant les revendications 1 ou 5.

**8.** Procédé de préparation de compositions pesticides, caractérisé en ce qu'on mélange des anilides d' acides cycloalkyl- ou hétérocyclyl-carboxyliques substitués de formule (I) suivant les revendications 1 ou 5 avec des diluants et/ou des agents tensioactifs.